# EUROPEAN PATENT APPLICATION

(11) **EP 4 122 509 A1**
(43) Date of publication of application: **25.01.2023**
(21) Application number: 22186310.3
(22) Date of filing: 21.07.2022
(51) Int. Cl.: A61M 3/02

(54) **DISPOSABLE DEVICE FOR INTIMATE HYGIENE**

(30) Priority: 23.07.2021 BR 102021014513
(71) Applicant: Mix For You Ltda., 11045-002 Santos SP (BR)
(72) Inventor: Castropil Logarzo, Marcus Augustus, SP 05417-000 São Paulo (BR)
(74) Representative: Fezzardi, Antonio

(57) **Abstract**

The device comprises two elements, being a flexible and collapsible casing (2) and a straight and fixed applicator nozzle (3) with a lid (4), fixed by pressure, the casing (2) equipped with an internal capacity of 300 ml, the casing (2) sealed along its perimeter by means of fusion by temperature and/or ultrasound, being further anatomical and presenting one of its sides (L1) curvilinear in a convex way and the other side (L2) presents small ripples (6) for the fingers; the straight and fixed applicator nozzle (3) of the device (1) is fixed to the casing (2) through pressure and has a dimension of 60 mm, whereby the fixed applicator nozzle (3) of the device (1) is presented structured as a long and smooth tubular element (7), on whose base (5) a plurality of circular transverse elements (8) are provided, parallel to each other, designed as means of bracing, presenting a larger diameter than the applicator tubular element (7).

## Description

### FIELD OF APPLICATION

This patent specification refers, more particularly, to a new device for intimate hygiene of single use or disposable and of easy portability as it presents a flexible and collapsible body, presented in an innovative way and endowed with features that personalize it regarding the devices provided for in the state of the art, providing its users with greater protection, hygiene and comfort, therefore, belonging to the field of medicinal syringes, such as, for example, irrigators or the like.

### PREAMBLE

This patent specification refers to a disposable device, developed for intimate hygiene, capable of promoting greater protection, safety, and well-being to the user, in addition to full hygiene, being easy and comfortable to use due to its ergonomic anatomy and dimension, providing greater ease of hygiene.

### STATE OF THE ART

The vast majority of people who seek to conduct intimate hygiene, whether vaginal or anal, have some solutions available in the consumer market that are not always the healthiest or most effective solutions.

Many people still use the conventional douches of showers to promote their intimate hygiene or even use hygiene devices.

There are devices called intimate vaginal douches, pear- or horn-shaped, which are defined by flexible rubber bulbs or pouches connected to a nozzle for conducting the hygiene.

Enemas are also provided for intestinal lavage, purging or drug administration through a rectal tube, being indicated for the diagnosis of rectal polyps and inflammatory bowel diseases, these consisting of a rubber pouch and/or plastic bags connected to hoses for the instillation of the liquid.

In addition, most of said douches or enemas are not comfortable, in addition to not being disposable.

Such intimate douches are essentially intended for vaginal use; however, they have often been used to induce the stimulation of peristaltic movements, which results in the excretion of residual feces, which is not the intended use for such a device.

Hygiene through shower douches or the like, due to the exacerbated pressure, perform a much deeper cleaning and, consequently, unnecessary, and may harm the mucosa of the anal region.

In case of anal intimate hygiene, the water pressure must not be strong, it must be at room temperature and, in addition, the device to be used must be disposable and the cleaning must only be in the rectal canal, so that it does not alter the mucosa of the region.

Vaginal intimate hygiene must be done only on the outside, that is, labia majora and labia minora, and close to the outside area, because if the cleaning is deeper, there may be unwanted changes in its natural flora, facilitating the emergence of infections.

In order to try to solve such inconveniences, the applicant of this application, always interested in advancing the development of its products, in order to provide the consumer public with new and quality products, already has another application with INPI, filed under the number BR 202014004079-5, on 02/21/2014, which refers, more particularly, to an intimate douche, which comprises a pouch (100) and a fixed (200) or screwable applicator nozzle.

In addition, just as an example, the current state of the art also includes the prior art document US2004-0118710A1, to Bourque et al., 2004, which discloses a device provided with a previously filled and sealed pouch, where the consumer does not have the option to choose the fluids to be used in their hygiene.

This device represents a flexible pouch with multiple chambers, with a frangible seal between them. It shows a standing pouch with two internal compartments, formed through one or two sheets of polymeric film sealed to each other, directly or indirectly through a third intervening polymeric film, said compartments existing so that the mixture of two ingredients can be done by the end user, shortly before consumption.

In addition, it features a frangible seal internal to the perimeter of the pouch, said seal dividing the pouch into a plurality of closed areas. It also provides for a liquid flavoring concentrate in one of said compartments and another ingredient confined to a separate second compartment.

The pouch provided for this device delaminates after sustained manual compression, producing a pressure increase within the separate compartment, confining the liquid.

The liquid applicator nozzle is formed by a thread section and the pouch does not have ergonomic characteristics for handling and application.

This document was added to the prior art list due to the format of the pouch. As it refers to ergonomics, it became important to mention it. This pouch features a design that fits the shape of a hand, making it easy to squeeze.

Several other documents cited as state of the art for this disposable device for intimate hygiene will be listed below, where the important intersections will be highlighted in each of them.

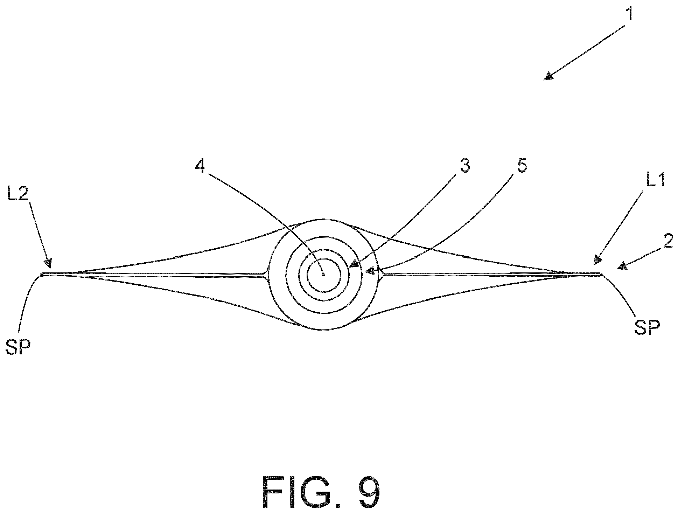

Document US6190366B1, to Tani, 2001 and CN2506189Y, claims a portable intimate douche comprising a pouch and an applicator nozzle, describing the use with water only and being performed with the use of a single hand (claim 1 and Fig. 9).

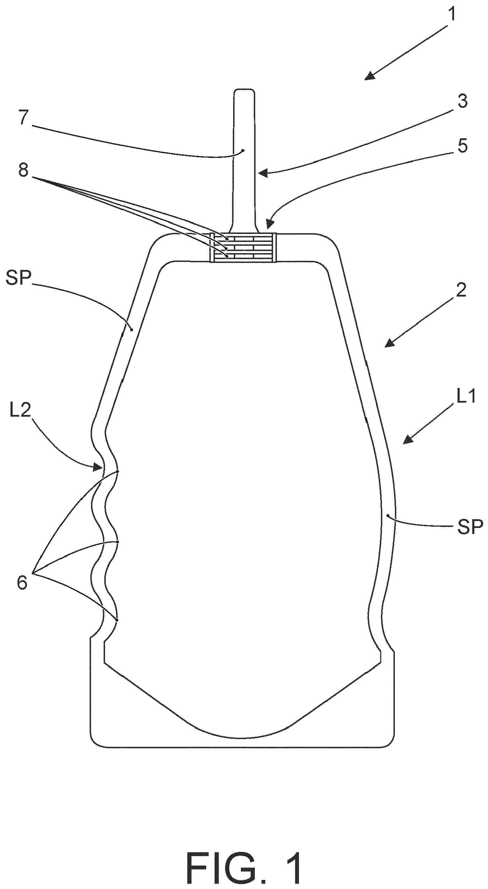

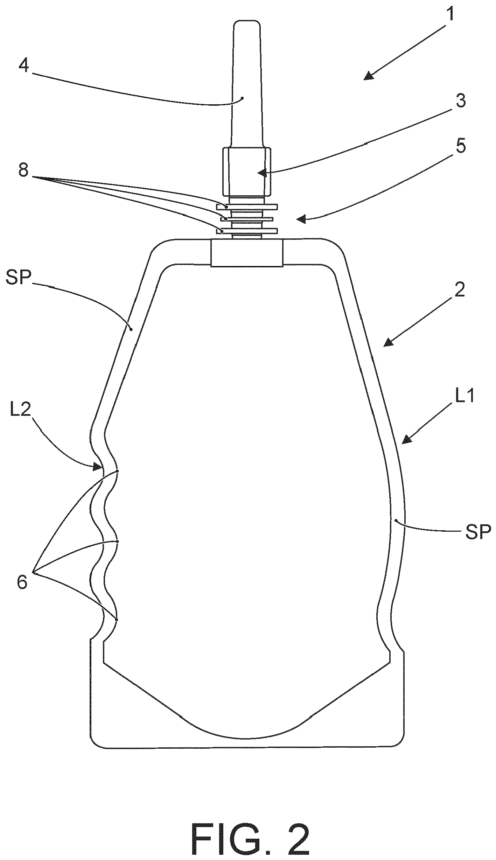

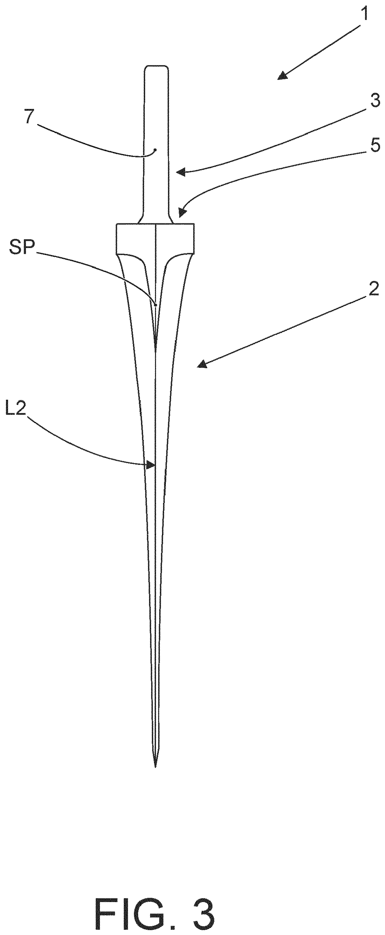

In this document, different possibilities are presented for the water outlet jet (E1, E2, and E3), including one with a single hole. Constructive variations are also featured, including one with indentations on the fringe of the pouch ("wave-shaped fringe") to prevent the wearer from getting cut. The feature that allows the object to stand is not highlighted. However, Figures 1 to 3 show a system that can stand up thanks to the valve, which keeps the water pressurized, and to the inner portion of the applicator nozzle (C), which keeps the nozzle tip facing upwards.

Another document found is US4180072, to Sneider, 1979 and GB1262926A, 1972, which describes a vaginal douche composed of a pouch and tube, with a manipulative cutoff valve. This previous document addresses housing in outer packaging.

In turn, document US4159718 A, to Bower, 1979, discloses a disposable douche composed of two plastic films thermally sealed at the ends and a tube.

Document US3844284, to Schoenfeld, 1974, shows a disposable douche with an accordion construction. This equipment has major differences; however, it was brought as a state-of-the-art douche capable of standing up.

Pior art document US3530858, to Edwards, 1970, shows a pouch and an applicator tube, whose assembly is done with an elastic band.

Another document pointed out is US3144866, to Ellis, 1964, with reveals in its text a pouch and a screwable applicator tube.

Document US3474788, to Corbin, 1969, discloses a disposable intimate douche with pouch and tube.

Likewise, document US3371665A, to Druckenmiller, 1968, discloses a disposable vaginal douche.

Document US3474936, to McDonnell, 1969, describes a liquid dispenser, where similarity with corrugations was pointed out, designed to allow selective flexibility in the rigid container, in order to obtain a structured deformation and a quick restoration.

The hygienic douche shown in the Brazilian document MU8102424, to Geisweller, 2001, is composed of an applicator and a tube for using an aqueous solution with a specific composition. Its physical conformation suggests rigid plastic parts, a threaded connection, and a container capable of standing.

Document US2005187526A1, to Horne, shows a portable bidet, presenting a very broad description, citing the use with a single hand, squeezing the container to force the fluid contained therein to escape.

Another document found is US5307955, to Viegas, 1994, presenting a flexible package with an opening at the bottom and a normally closed valve. Said document refers to a pouch for fluid content and, in the application, the use with products such as shampoo, conditioner, soaps, detergents and the like is described. This is a relevant document in that it has the same pouch format and claims to stand up ("stand up base", "stand alone on a flat surface").

Document USD474683S, to Berman, 2003, refers to an industrial design of a beverage pouch. It is listed in the European Union process for having the same shape as the pouch and, therefore, capable to stand up.

Finally, in addition to all these documents cited, it is also possible to highlight some uncited documents, however relevant, such as the Chinese prior art document, CN2113726U, which shows a douche with a pouch and a tube in packaging.

Thus, from all these considerations, it is understood that there are still many inconveniences that occur due to the use of conventional intimate douches and even conventional sanitizers found in the similar market.

### BRIEF DESCRIPTION OF THE INNOVATION

In order to overcome the drawbacks known to the state of the art and also to take an evolutionary step in its previous patent application, BR 202014004079-5, the holder has developed a practical, anatomical, portable, and disposable intimate douche, of easy portability, especially for women's intimate hygiene and also for use as an enema and pelvic cavities for women and men.

Thus, it created and developed this disposable device for intimate hygiene, which must be positioned with complete prominence among its congeners and be customized to the consumer market by presenting a device for intimate hygiene, portable and disposable, basically composed of two elements, being a flexible, collapsible casing for the containment of liquids, such as water or medicines for hygiene, and an applicator nozzle with a lid, which is fixed to said casing through pressure.

Said intimate sanitizer is capable of providing fast and adequate hygiene, without excessive water pressure, a fact that is likely to cause inconvenience to the user and can be used as enemas for intestinal and other treatments, being usable in female and male pelvic cavities, injecting a flow of water or other related fluid, in order to assist the user in treatments, flora replacement, adequate lubrication of said cavities.

The main object of this patent application is, therefore, to provide a multitasking, disposable sanitizer that is capable of providing the user with full comfort, safety, and effectiveness in its use.

### BRIEF DESCRIPTION OF THE DRAWINGS

In order to complement this description and obtain a better understanding of the characteristics of the patent and in accordance with a preferred practical embodiment thereof, the attached description is accompanied by a set of drawings, where, in an exemplified way, although not limiting, the following was represented:
Figure 1 shows a front plan view of the disposable device for intimate hygiene with fixed nozzle of this patent;
Figure 2 shows a front plan view of the disposable device for intimate hygiene with removable nozzle;
Figure 3 shows a side view of the empty disposable device with fixed nozzle;

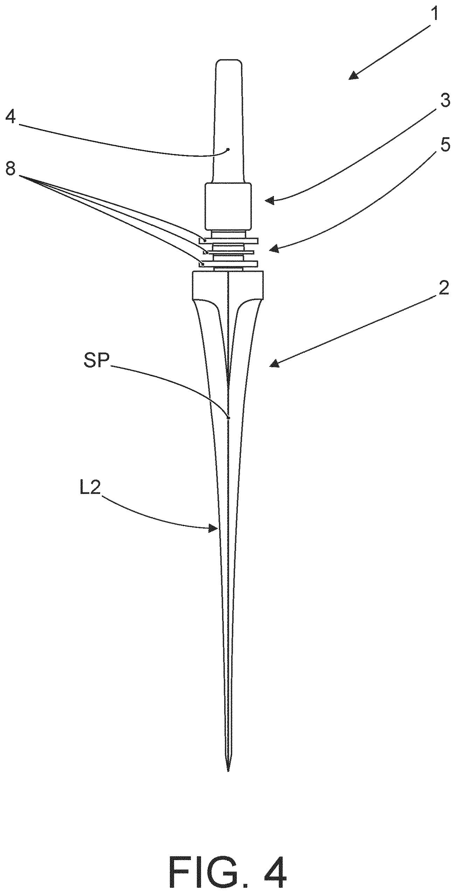

Figure 4 shows a side view of the empty disposable device with removable nozzle;

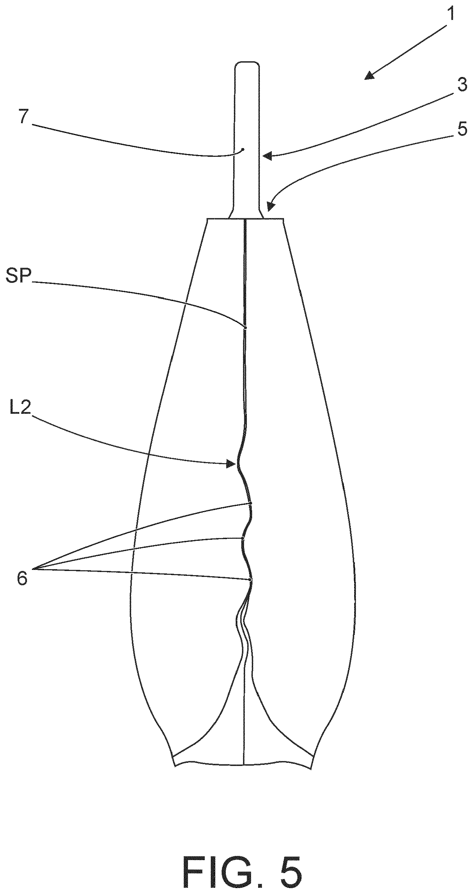

Figure 5 is a side view of the disposable device for intimate hygiene with fixed nozzle in its filled and ready-to-use state;
Figure 6 is a side view of the disposable device for intimate hygiene with removable nozzle in its filled and ready-to-use state;

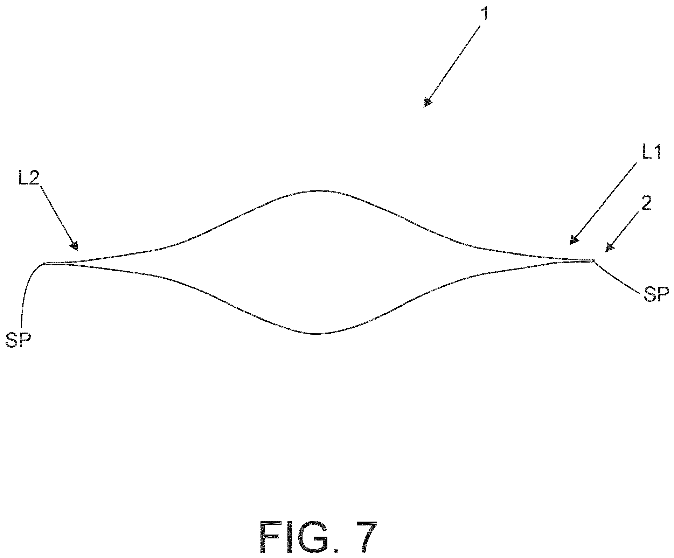

Figure 7 shows a bottom view of the disposable device for intimate hygiene in its filled and ready-to-use state;

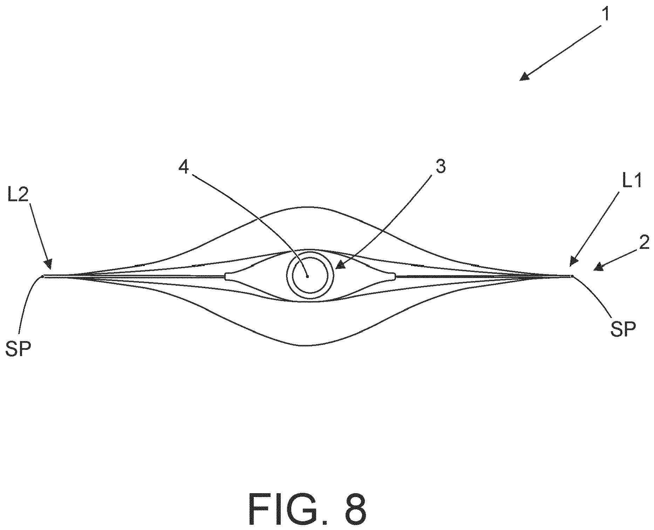

Figure 8 is a top view of the disposable device for intimate hygiene with a fixed nozzle in its filled and ready-to-use state;
Figure 9 shows a top view of the disposable device for intimate hygiene with removable nozzle in its filled and ready-to-use state;

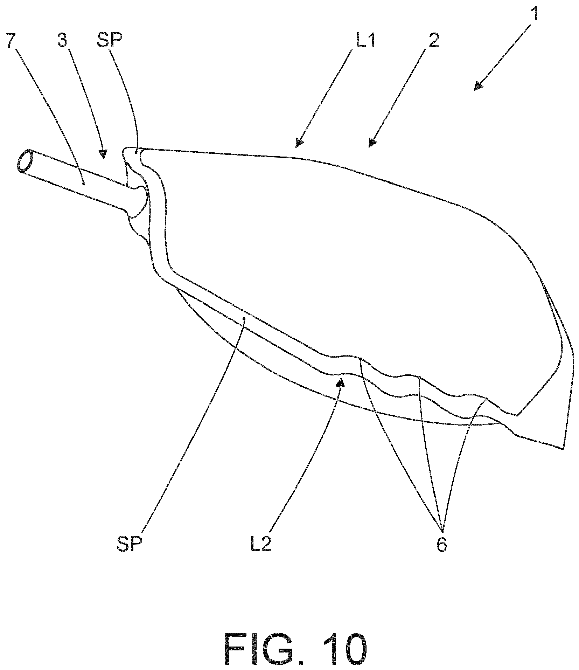

Figure 10 is a perspective view of the disposable device for intimate hygiene with fixed nozzle in its filled and ready-to-use state; and

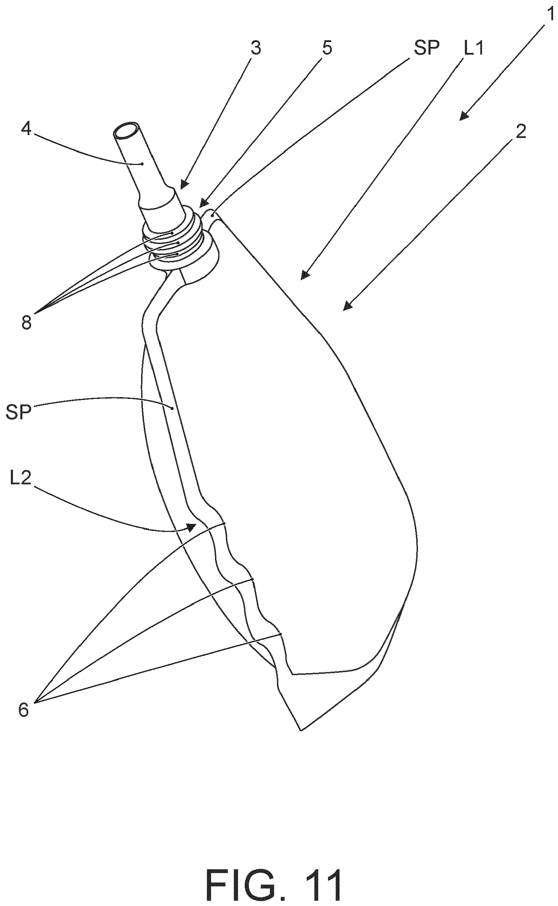

Figure 11 shows a perspective view of the disposable device for intimate hygiene with removable nozzle in its filled and ready-to-use state.

### DETAILED DESCRIPTION OF THE INVENTION

According to what illustrated by the above-mentioned figures, this patent relating to a disposable device for intimate hygiene, presents a portable, collapsible, and disposable device 1 for intimate hygiene, basically composed of two elements, one being a flexible and collapsible casing 2 and the other a straight and fixed applicator nozzle 3 with a lid 4, the nozzle 3 being fixed by pressure.

The casing 2 or liquid enclosure is defined as intestinal flora replenishers, anesthetics, lubricants, water for cleaning, among others.

The casing 2 of this device 1 contains an internal capacity, variable in volume between 50 and 800 ml, preferably marketable with a volume of 300 ml, made from a multilayer polymeric film, preferably polyethylene terephthalate (PET), laminated with polyethylene of low density and/or bioriented nylon films, BOPP, PP, nylon/poly films, among others, sealed along its perimeter by means of temperature fusion and/or ultrasound, so as to form the aforementioned casing 2 of the device 1 in question.

The perimeter sealing SP of this casing excludes the need to use reinforcements at the junction with the base 5 of the applicator nozzle 3.

The housing 2 of the disposable device 1 for intimate hygiene is anatomical, presenting one of its sides L1 curvilinear in a convex way and the other side L2 presents small ripples 6 for the perfect fit of the fingers of the hand, providing a precise grip with total security in the application.

The fixed applicator nozzle 3 of the device 1 for hygiene is fixed to said casing 2 through pressure and has a dimension that can vary between 30mm and 150mm, being preferably marketable with 60mm.

For this purpose, the fixed applicator nozzle 3 of said device 1 for intimate hygiene is structured as a long and smooth tubular element 7, on whose base 5 a plurality of circular transverse elements 8 are provided, parallel to each other, designed as means of bracing, presenting a larger diameter than the applicator tubular element, thus allowing a perfect junction between the perimeter band of the casing and the aforementioned fixed applicator nozzle, eliminating the need for structural reinforcements to prevent possible leaks in its use.

From the physical and structural conformation of the disposable device for intimate hygiene, it becomes possible to use this device in the application of water for hygiene, in addition to other products for the various treatments in the region, that is, gels, powders, granules, vaginal tablets, suppositories, among other products for the interior of the female and/or male pelvic cavity in order to assist in the hygiene, treatment, flora replacement, lubrication and/or anesthesia of such cavities.

In order to use the disposable device for intimate hygiene, the casing must be filled up to the indicated limit, fixing the applicator nozzle and then performing the application in the pelvic cavity of interest, manually pressing the content into it.

For application in the vaginal cavity, preferably the user should use pure water to clean the area, eliminating fluids such as menstrual residues and other secretions.

Other products, such as medicines indicated for treatments in the region, can be applied in the vaginal cavity.

In turn, when using the device for hygiene of the anal cavity, one can obtain a cleaning from the distal portion of the large intestine, rectum, and anal canal.

The cleaning procedure performed through the disposable device for intimate hygiene must be performed two to three times to obtain the expected result.

When used in the anal cavity, it provides the stimulus of peristaltic movements in the sigmoid, which is the interconnection of the large intestine and rectum, by the contact of water in the pectineal line, so that all fecal residues are purged.

As it is a single-use device, after use, the user must discard the device, in order to preserve their health.

The disposable device for intimate hygiene has a substantial structural improvement in design compared to the previous ones, and said device, once opened, is capable of holding large internal volumes of useful fluid and, when closed, occupies a minimum volume.

By defining a ratio between these two volumes, that is, the capacity of the product over the volume of the packaged product, it is possible to visualize one of the advantages of the disposable device for intimate hygiene, object of this application, for allowing the use of large volumes per appliance.

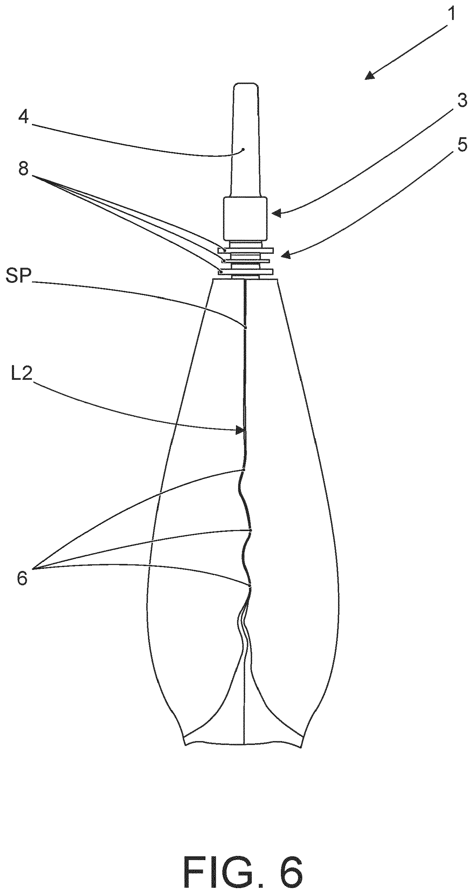

Said disposable device for intimate hygiene stands up due to the shape of the pouch (Fig. 6), presenting three polymeric films joined together, creating this specific configuration of the fringe without depending on a valve, such as the related devices of the state of the art do. This is a physical characteristic from which an important functionality derives, which is to provide the user with freedom with their hands when preparing to use the device, leaving the device standing until it is ready to use it.

Another very important aspect is the ergonomics embedded in the context of this device for intimate hygiene. The anatomic ridges or ripples of the fringe are designed to accommodate one hand, allowing for a more effective transmission of force when squeezing the product. Ultimately, this allows the user to handle a greater volume of internal content.

The outlet channel of the fixed applicator nozzle has a single large diameter hole for exiting the contents of the casing, enabling large flows for low-viscosity fluids, and enabling high-viscosity fluids and solid dosage forms such as powders, granules, tablets, suppositories, etc.

Finally, as it has only one rigid element, namely, the fixed applicator nozzle, this device provides a great decrease in the volume of the packaged product, also due to the fact that the casing is folded and housed in packaging of minimum dimensions, greatly optimizing its portability.

While the preferred embodiment of this patent is described, any changes and/or modifications must be understood as within the scope of the patent, perfectly fitting the criteria that define it, that is, the combination and modification of elements already known in a new form or arrangement, resulting in a functional improvement in their use or in their manufacture.

## Claims

1. Disposable device for intimate hygiene, wherein said device (1) basically comprises two elements, being a flexible and collapsible casing (2) when empty, and a straight and fixed applicator nozzle (3) with a lid (4), fixed by pressure; the casing (2) contains an internal capacity varying in volume between 50 and 800 ml, preferably marketable with a volume of 300 ml, made from a multilayer polymeric film, from the group consisting of one of these elements: polyethylene terephthalate (PET), laminated with low density polyethylene, bioriented nylon films, BOPP, PP, nylon/poly films; the casing (2) is sealed along its perimeter using one of the techniques from the following group: temperature melting, ultrasound; the casing (2) of the device (1) is anatomical, presenting one of its sides (L1) curvilinear in a convex way and the other side (L2) presents small ripples (6) for the fingers; the straight and fixed applicator nozzle (3) of the device (1) is fixed to the casing (2) through pressure and has a variable dimension between 30mm and 150mm.

2. Disposable device for intimate hygiene according to claim 1, wherein said casing (2) has a dimension of 60mm.

3. Disposable device for intimate hygiene according to claim 1, wherein the fixed applicator nozzle (3) of the device (1) is structured as a long and smooth tubular element (7), on whose base (5) a plurality of circular transverse elements (8) are provided, parallel to each other, designed as means of bracing, having a larger diameter than the applicator tubular element (7).

4. Disposable device for intimate hygiene according to claim 1, wherein the applicator nozzle (3) provides an outlet channel that has a single hole of large diameter, for the exit of the contents of the casing, enabling large flow rates for low-viscosity fluids and enabling high-viscosity fluids and solid dosage forms such as powders, granules, tablets, suppositories, etc.
